# EUROPEAN PATENT APPLICATION

(11) **EP 2 527 327 A1**
(43) Date of publication of application: **28.11.2012**
(21) Application number: 11734728.6
(22) Date of filing: 20.01.2011
(51) Int. Cl.: C07D 213/73

(54) **METHOD FOR PRODUCING 2-AMINO-4-(TRIFLUOROMETHYL)PYRIDINE**

(30) Priority: 21.01.2010 JP 2010010890
(71) Applicant: Ishihara Sangyo Kaisha, Ltd., Osaka-shi, Osaka 550-0002 (JP)
(72) Inventor: OKADA Takashi, Shiga 525-0025 (JP)
(74) Representative: HOFFMANN EITLE
(86) International application number: PCT/JP2011/050996
(87) International publication number: WO 2011/090122

(57) **Abstract**

The present invention relates to a process for producing a 2-amino-4-trifluoromethylpyridine. Specifically, the present invention provides a process for producing a compound represented by the formula (I) or a salt thereof: 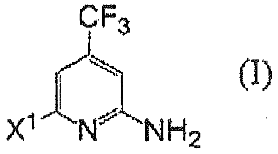 (in the formula, X¹ is the same as X¹ in the formula (II) below), which includes allowing a compound represented by the formula (II): 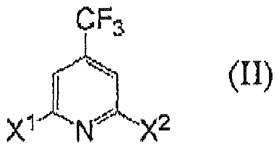 (in the formula, each of X¹ and X² is independently a chlorine atom or a bromine atom) to react with ammonia in the presence of a hydrophilic ether; and also a process for producing 2-amino-4-trifluoromethyl pyridine or a salt thereof, which comprises subjecting the compound of the formula (I) as produced by the foregoing process to dehalogenation,

## Description

### [TECHNICAL FIELD]

The present invention relates to a process for producing 2-amino-4-trifluoromethylpyridines which are useful as intermediates for pharmaceuticals or agrochemicals.

### [BACKGROUND ART]

As a production process of 2-amino-4-trifluoromethylpyridines, for example, a process for producing 2-amino-6-chloro-4-trifluoromethylpyridine by allowing 2,6-dichloro-4-trifluoromethylpyridine to react with ammonia is known (Patent Document 1). In addition, a process for producing 2-amino-4-trifluoromethylpyridine by allowing 2-chloro-4-trifluoromethylpyridine to react with ammonia is known (Patent Document 2 and Non-Patent Document 1).
However, from the standpoint of industrial production, the conventional processes have such problems that the reactivity is not sufficient, a long-time reaction is required, a proportion of by-products is high, and the like.

### [CITATION LIST]

### [PATENT DOCUMENTS]

Patent Document 1: JP-A-63-48268
Patent Document 2: European Patent No. 228846

### [NON-PATENT DOCUMENT]

Non-Patent Document 1: Journal of Fluorine Chemistry (1999),93, 153 to 157

### [SUMMARY OF THE INVENTION]

### [PROBLEM TO BE SOLVED BY THE INVENTION]

A process for producing 2-amino-4-trifluoromethylpyridines in a high purity for a short period of time is provided.

### [MEANS FOR SOLVING THE PROBLEM]

As a result of extensive and intensive investigations for the purpose of solving the foregoing problem, a process in which a compound represented by the formula (II):

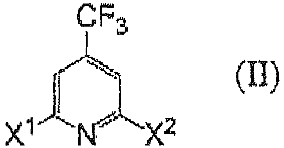

(in the formula, each of X¹ and X² independently represents a chlorine atom or a bromine atom) is allowed to react with ammonia in the presence of a hydrophilic ether, thereby producing a compound represented by the formula (I):

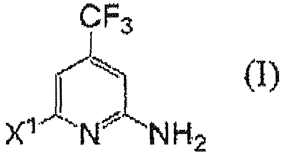

(in the formula, X¹ is the same as X¹ in the formula (II)) in a high purity for a short period of time, has been found. Furthermore, a process in which the obtained compound of the formula (I) is subjected to dehalogenation, thereby producing 2-amino-4-trifluoromethylpyridine in a high purity for a short period of time, has been found.
Thus, the present invention relates to a process for producing a compound of the formula (I) or a salt thereof comprising allowing a compound of the formula (II) to react with ammonia in the presence of a hydrophilic ether; and also relates to a process for producing 2-amino-4-trifluoromethylpyridine or a salt thereof comprising subjecting the compound of the formula (1) as produced by the foregoing process to dehalogenation.

### [EFFECT OF THE INVENTION]

According to the production process of the present invention, 2-amino-4-trfluoromethylpyridines can be produced under industrially advantageous conditions.

### [EMBODIMENTS FOR CARRYING OUT THE INVENTION]

As shown in the following Reaction (A), the compound of the formula (I) can be produced by allowing the compound of the formula (II) to react with ammonia in the presence or a hydrophilic ether.

Reaction (A):

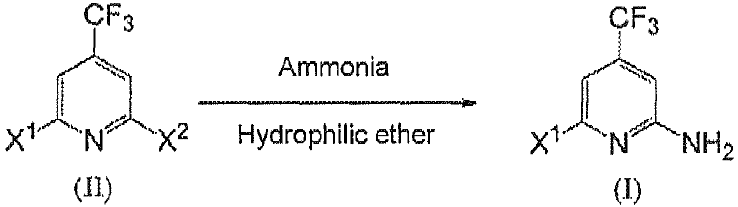

(In the formulae (I) and (II), X¹ and X² are the same as described above.)
Examples of the salt of the compound of the formula (I) include acid addition salts and the like. Examples of the acid addition salt include acid addition salts such as hydrochlorides and sulfates. The salt of the compound of the formula (I) can be produced in accordance with a known salt forming reaction.
X¹ and X² are preferably a chlorine atom.
Examples of ammonia include ammonia water and ammonia gas.
Ammonia can be used in an amount of preferably from 3 to 15 times by mole, and more preferably from 5 to 12 times by mole per one mole of the compound of the formula (11). However, an amount falling outside this range can also be adopted, depending upon reaction conditions.

The hydrophilic ether is not particularly limited so far as it is inert to the reaction. Examples of the hydrophilic ether include cyclic ethers such as tetrahydrofuran, 2-methyltetrahydrofuran, 3-methyltetrahydrofuran, and 1,4-dioxane; straight-chain ethers such as 1,2-dimethoxyethane; and the like. As the hydrophilic ether, for example, tetrahydrofuran, 2-methyltetrahydrofuran, 3-methyltetrahydrofuran, 1,2-dimethoxyethane, and the like are preferable.

The hydrophilic ether can be used in an amount of preferably from 1 to 10 times by volume (V/W), and more preferably from 1 to 5 times by volume (V/W) relative to the weight of the compound of the formula (II). However, an amount falling outside this range can also be adopted, depending upon reaction conditions.

The present reaction can also be conducted in the presence of a solvent. The solvent is not particularly limited so far as it is inert to the reaction. Examples of the solvent include aliphatic hydrocarbons such as pentane, hexane, heptane, octane, and cyclohexane; aromatic hydrocarbons such as benzene, toluene, and xylene; ethers such as diethyl ether, butyl ethyl ether, and methyl tert-butyl ether; polar aprotic solvents such as acetonitrile, propionitrile, N,N-dimethylformamide, dimethyl sulfoxide, hexamethylphosphoric triamide, sulfolane, N,N-dimethylacetamide, and N-methylpyrrolidone; esters such as methyl acetate, ethyl acetate, and propyl acetate; alcohols such as methanol, ethanol, propanol, n-butanol, and tert-butanol; water; and the like.

A reaction temperature is preferably from about 100 to about 200 °C, and more preferably from about 130 to about 160 °C; and a reaction time is preferably from about 2 to about 24 hours, and more preferably from about 4 to about 7 hours.

As shown in the following Reaction (B), 2-amino-4-trifluoromethylpyridine can be produced by allowing the compound of the formula (II) to react with ammonia in the presence of a hydrophilic ether to produce the compound of the formula (I) and subsequently subjecting the obtained compound of the formula (I) to dehalogenation.

Reaction (B):

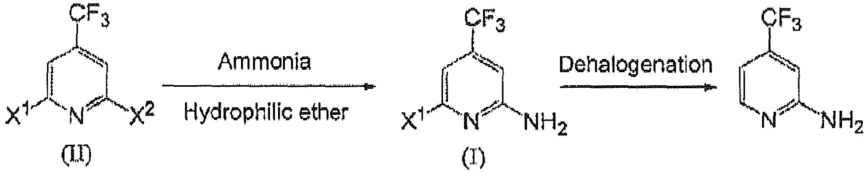

(In the formulae (I) and (II), X¹ and X² have the same meanings as those in X¹ and X² in the Reaction (A).)
In the foregoing reaction, the dehalogenation reaction can be conducted without isolating or purifying the compound of the formula (I).
In addition, examples of the salt of 2-amino-4-trifluoromethylpyridine include acid addition salts and the like. Examples of the acid addition salt include hydrochlorides, sulfates, and the like. The salt of 2-amino-4-trifluoromethylpyridine can be produced in accordance with a known salt forming reaction.
X¹ and X² are preferably a chlorine atom.
The reaction between the compound of the formula (II) and ammonia can be conducted in the same manner as that in the foregoing Reaction (A). However, since ammonia can also serve as a base in the dehalogenation step, its use amount may be increased, if desired. In the case of increasing the use amount of ammonia, the amount can be set to preferably from 3 to 20 times by mole, and more preferably from 5 to 15 times by mole per one mole of the compound of the formula (II). However, an amount falling outside this range can also be adopted, depending upon reaction conditions.

The dehalogenation can be, for example, conducted by means of catalytic reduction. In general, the catalytic reduction can be conducted by allowing the desired compound to react with a hydrogen source in the presence of a catalyst and a solvent. Examples of the hydrogen source include hydrogen, ammonium formate, formic acid, triethylammonium formate, and sodium hypophosphite; mixtures of two or more members properly selected from these compounds; and the like. Of these, hydrogen is preferable as the hydrogen source. Examples of the catalyst include platinum, platinum oxide, platinum black, Raney nickel, palladium, palladium-carbon, rhodium, rhodium-alumina, ruthenium, iron, copper, and the like. Of these, palladium-carbon and the like are desirable as the catalyst. Incidentally, the catalyst may be used alone or in combination of two or more kinds thereof.

The hydrogen source can be used in an amount of preferably from 1 to 10 times by mole, and more preferably from 1 to 5 times by mole per one mole of the compound of the formula (I). In addition, the catalyst can be used in an amount of preferably from 0.01 to 1 % by mole, and more preferably from 0.05 to 0.5 % by mole per one mole of the compound of the formula (I). However, an amount falling outside those ranges can also be adopted, depending upon reaction conditions. Incidentally, in the case of conducting the dehalogenation without isolating or purifying the compound of the formula (I), the hydrogen source or the catalyst may be used in the same proportion as described above per one mole of the compound of the formula (II).

A solvent which is usually used for catalytic reduction can be used as the solvent Examples of the solvent include the same solvents as those in the foregoing step. In the case of conducting the catalytic reduction without isolating or purifying the compound of the formula (I), the solvent used in the reaction between the compound of the formula (II) and ammonia can be used as it is.

In general, the dehalogenation is conducted in the presence of a base. Examples of the base include ammonia; alkali metal hydroxides such as sodium hydroxide and potassium hydroxide; alkali metal carbonates such as sodium carbonate and potassium carbonate; alkali metal bicarbonates such as sodium bicarbonate and potassium bicarbonate; tertiary amines such as trimethylamine, triethylamine, triisopropylamine, diisopropylethylamine, pyridine, 4-dimethylaminopyridine, 2,6-dimethylpyridine, 4-pyrrolidinopyridine, N-methylmorpholine, NN-dimethylaniline, N,N-diethylaniline, N-ethyl-N-methylaniline, 1,8-diazabicyclo[5.4.0]-7-undecene, and 1,4-diazabicyclo[2.2.2]octane; and the like. Above all, by making ammonia used in the preceding step also serve as the base, the reaction can be efficiently conducted.

The base can be used in an amount of preferably from 1 to 2 equivalents, and more preferably from 1 to 1.5 equivalents per one mole of the compound of the formula (I). However, an amount falling outside this range can also be adopted, depending upon reaction conditions. Incidentally, in the case of conducting the dehalogenation without isolating or purifying the compound of the formula (I), the base may also be used in the same proportion as described above per one mole of the compound of the formula (II).
A reaction temperature of the dehalogenation is preferably from about 20 to about 150 °C, and more preferably from about 60 to about 130 °C; and a reaction time is preferably from about 0.5 to about 10 hours, and more preferably from about 1 to about 5 hours.

Preferred embodiments of the present invention are hereunder described, but it should not be construed that the present invention is limited thereto.
(1) A process for producing 2-amino-6-chloro-4-trifluoromethylpyridine or a salt thereof comprising allowing 2,6-dichloro-4-trifluoromethylpyridine to react with ammonia in the presence of a hydrophilic ether.
(2) A process for producing 2-amino4-trifluoromethylpyridine or a salt thereof comprising allowing 2,6-dichloro-4-trifluoromethylpyridine to react with ammonia in the presence of a hydrophilic ether to produce 2-amino-6-chloro-4-trifluoromethylpyridine and subsequently subjecting the obtained 2-amino-6-chloro-4-trifluoromethylpyridine to dehalogenation.
(3) The process as set forth above in (1) or (2), wherein the hydrophilic ether is at least one member selected from the group consisting of tetrahydrofuran, 2-methyltetrahydrofuran, 3-methyltetrahydrofuran, and 1,2-dimethoxyethane.
(4) The process as set forth above in (1) or (2), wherein the hydrophilic ether is at least one member selected from the group consisting of tetrahydrofuran and 1,2-dimethoxyethane.
(5) The process as set forth above in (1) or (2), wherein the hydrophilic ether is used in an amount of from 1 to 10 times (V/W) relative to the 2,6-dichloro-4-trifluoromethylpyridine.

### [EXAMPLES]

In order to describe the present invention in more detail, Examples are described below, but it should not be construed that the present invention is limited thereto. Abbreviations in each of the synthesis examples and each of the tables are as follows.
2,6,4-DCTF: 2,6-Dichloro-4-trifluoromethylpyridine
2,6,4-ACTF: 2-Amino-6-chloro-4-trifluorometlrylpyridine
2,6,4-DATF: 2,6-Diamino-4-trifluoromethylpyridine
2,4-ATF: 2-Amino-4-trifluoromethylpyridine
THF: Tetrahydrofuran
2-Me-THF: 2-Methyltetrahydrofuran
DME: 1,2-Dimethoxyethane
Pd/C: Falladium-carbon
HPLC-PA % in each of the tables empresses a peak area rate (peak area %) analyzed by high performance liquid chromatography (HPLC). The measuring conditions are as follows.
Column: Waters 5C19-AR φ4.6 mm x 150 mm
Mobile phase: Methanol/water = 4/1, 0.5 % acetic acid
Column temperature: 40 °C
Flow rate: 1 mL/min
Detection: 254 nm

### Example 1

In an autoclave (100 mL), 5 g (0.023 moles) of 2,6,4-DCTF, 15.3 mL (0.22 moles) of 28 % ammonia water, and 10 mL of THF were heated to 150°C with stirring to effect reaction. After about 5 hours, the autoclave was cooled to from 30 to 40 °C. Water was added to the reaction solution; the extraction with ethyl acetate was conducted three times; and an organic layer was washed with saturated saline and then dried over sodium sulfate. The organic layer was concentrated under reduced pressure to obtain 2,6,4-ACTF.

### Comparative Example 1

2,6,4-ACTF was obtained by conducting the reaction in the same manner as that in Example 1, except for not using THF.

As for each of Example 1 and Comparative Example 1, the reaction solution was analyzed by high performance liquid chromatography (HPLC). HPLC-PA % of each of 2,6,4-DCTF as a raw material compound, 2,6,4-ACTF as a desired compound, and 2,6,4-DATF as a by-product is described in Table 1.

**Table 1**

| | hydrophilic ether | HPLC-PA% | | |
|---|---|---|---|---|
| | | 2,6,4-DCTF | 2,6,4-ACTF | 2,6,4-DATF |
| Example 1 | THF | 2.5 | 94.5 | 1.5 |
| Comparative Example 1 | Nil | 24.1 | 72.5 | 1.8 |

### Example 2

In an autoclave (100 mL), 5 g (0.023 moles) of 2,6,4-DCTF, 15.4 mL (0.22 moles) of 28 % ammonia water, and 13 mL of THF were heated to 150 °C with stirring to effect reaction. After about 5 hours, the autoclave was cooled to from 30 to 40 °C. 150 mg of 5 % Pd/C (54 % wet, (1.038 mmoles) was added thereto, hydrogen was filled up to 2.0 MPa, and the contents were heated to 100 °C with stirring to effect reaction. After about 3 hours, the autoclave was cooled to from 30 to 40 °C, followed by filtration through Celite. Water was added to a filtrate; the extraction with ethyl acetate was conducted three times; and an organic layer was washed with saturated saline and then dried over sodium sulfate. The organic layer was concentrated under reduced pressure, and n-hexane was added, followed by concentration under reduced pressure. A deposited crystal was dried under reduced pressure, thereby obtaining 3 g of 2,4-ATF as a white crystal (yield (crude): 79.9 %).

### Example 3

3.03 g of 2,4-ATF was obtained as a white crystal (yield (crude): 80.7 %) by conducting the reaction in the same manner as that in Example 2, except for using 15.3 mL (0.22 moles) of 28 % ammonia water and 10 mL of THF.

### Example 4

3.34 g of 2,4-ATF was obtained as a white crystal (yield (crude): 89.0 %) by conducting the reaction in the same manner as that in Example 3, except for changing THF to DME.

### Comparative Example 2

In an autoclave (100 mL), 5 g (0.023 moles) of 2,6,4-DCTF and 15.3 mL (0.22 moles) of 28 % ammonia water were heated to 150 °C with stirring to effect reaction. After about 15 hours, the autoclave was cooled to from 30 to 40 °C. 150 mg of 5 % Pd/C (54 % wet, 0.038 mmoles) was added thereto, hydrogen was filled up to 1.8 MPa, followed by heating to 100°C with stirring to effect reaction. After about 3 hours, the autoclave was cooled to from 30 to 40 °C, followed by filtration through Celite. Water was added to the filtrate; the extraction with ethyl acetate was conducted three times; and an organic layer was washed with saturated saline and then dried over sodium sulfate. The organic layer was concentrated under reduced pressure, and after adding n-hexane, followed by concentration under reduced pressure. A deposited crystal was dried under reduced pressure, thereby obtaining 3 g of 2,4-ATF as a pale ocher crystal (yield (crude): 79.9 %).

As for each of Examples 2, 3 and 4 and Comparative Example 2, the obtained crystal was analyzed by high performance liquid chromatography (HPLC). HPLC-PA % of each of 2,6,4-ACTF as an intermediate product, 2,4-ATF as a desired compound, and 2,6,4-DATF as a by-product and the yield of 2,4-ATF are described in Table 2.

**Table2**

| | hydrophilic ether | HPLC-PA% | | | Yield (%) |
|---|---|---|---|---|---|
| | | 2,6,4-ACTF | 2,4-ATF | 2,6,4-DATF | |
| Example 2 | THF | 1.4 | 92.9 | 3.4 | 79.9 |
| Example 3 | THF | 3.0 | 90.8 | 4.5 | 80.7 |
| Example 4 | DME | 0.6 | 94.7 | 3.9 | 89.0 |
| Comparative Example 2 | Nil | 2.5 | 81.1 | 9.7 | 79.9 |

### Example 5

In an autoclave (200 mL), 10 g (0.046 moles) of 2,6,4-DCTF, 30.6 mL (0.44 moles) of 28 % ammonia water, and 20 mL of THF were heated to 150 °C with stirring to effect reaction. After about 6 hours, the autoclave was cooled to from 30 to 40 °C, 300 mg of 5 % Pd/C (54 % wet, 0.076 mmoles) was added thereto, hydrogen was filled up to 2.0 MPa, followed by heating to 100 °C with stirring to effect reaction. After about 3 hours, the pressure reactor was cooled to from 30 to 40 °C, followed by filtration through Celite. Water was added to the filtrate; the extraction with ethyl acetate was conducted three times; and an organic layer was washed with saturated saline and then dried over sodium sulfate. The organic layer was concentrated under reduced pressure, and n-hexane was added, followed by concentration. To a deposited crystal, n-hexane was added, followed by stirring under ice cooling for about 60 minutes, and a deposited crystal was filtered. The obtained crystal was subjected to washing with ice-cooled n-hexane three times and then dried under reduced pressure to obtain 5.4 g of 2,4-ATF as a white crystal (yield: 71.9 %, melting point: 70.1 °C). In addition, an NMR spectrum data of the obtained crystal is as follows.
¹H-NMR (CDCl₃): δ 4.713 (brs, 2H), 6.684 (s, 1H), 6.823 (dd, J = 5.2 and 1.2 Hz, 1H), 8.205 (d, J = 5.6 Hz, 1H)

As for Example 5, the obtained white crystal was analyzed by high performance liquid chromatography (HPLC). HPLC-PA % of each of 2,6,4-ACTF as an intermediate product, 2,4-ATF as a desired compound, and 2,6,4-DATF as a by-product and the yield of 2,4-ATF are described in Table 3.

**Table3**

| | hydrophilic ether | HPLC-PA% | | | Yield (%) |
|---|---|---|---|---|---|
| | | 2,6,4-ACTF | 2,4-ATF | 2,6,4-DATF | |
| Example 5 | THF | 0.8 | 96.0 | 2.5 | 71.9 |

### Example 6

A hydrogen chloride gas was gradually blown into a mixture of 6 g of 2,4-ATF and 80 mL of dry toluene at from 10 to 20 °C while stirring, After stirring at room temperature for 30 minutes, filtration was conducted, and a crystal was subjected to washing with dry toluene three times. The obtained crystal was dried under reduced pressure to obtain 7.1 g of 2,4-ATF hydrochloride as a white crystal (yield: 96.6 %, melting point: 218 °C). In addition, an NMR spectrum data of the obtained crystal is as follows.
¹H-NMR (D₂O): δ 4.650 (brs, 2H), 6.957 (dd, J = 6.8 and 2.0 Hz, 1H), 7.209 (d, J = 0.8 Hz, 1H), 7.838 (d, J = 6.4 Hz, 1H)

### Example 7

In an autoclave (200 mL), 10 g (0.046 moles) of 2,6,4-DCTF, 30.6 mL (0.44 moles) of 28 % ammonia water, and 20 mL of 2-Me-THF were heated to 150 °C with stirring to effect reaction. After about 6 hours, the autoclave was cooled to from 30 to 40 °C. 300 mg of 5 % Pd/C (54 % wet, 0.076 mmoles) was added thereto, hydrogen was filled up to 1.6 MPa, followed by heating to 100 °C with stirring to effect reaction. After about 3 hours, the pressure reactor was cooled to from 30 to 40 °C, followed by filtration through Celite. Water was added to a filtrate, and only a separated organic layer was collected. An aqueous layer was extracted with ethyl acetate once, and the organic layer was collected. The collected organic layers were mixed, washed with saturated saline, and then dried over sodium sulfate. The organic layer was concentrated under reduced pressure, to which then n-hexane was added, followed by concentration. To a deposited crystal, n-hexane was added, followed by stirring under ice cooling for about 30 minutes, and a deposited crystal was filtered. The obtained crystal was subjected to washing with ice-cooled n-hexane three times and then dried under reduced pressure to obtain 5.36 g of 2,4-ATF as a white crystal (yield: 71.4 %).

While the invention has been described in detail and with reference to specific embodiments thereof, it will be apparent to one skilled in the art that various changes and modifications can be made therein without departing from the spirit and scope thereof.
Incidentally, the present application is based on a Japanese patent application filed on January 21, 2010 (Japanese Patent Application No. 2010-010890), the entirety of which is incorporated herein by reference.
In addition, the entire disclosures as referred to therein are incorporated herein by reference in its entirety.

### [INDUSTRIAL APPLICABILITY]

According to the production process of the present invention, 2-amino-4-trfluoromethylpyridines can be produced under industrially advantageous conditions.

## Claims

1. A process for producing a compound represented by the formula (I) or a salt thereof: 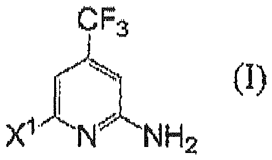 wherein X¹ is the same as X¹ in the formula (II) below,
which comprises allowing a compound represented by the formula (II): 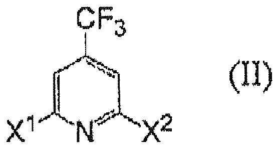 wherein each of X¹ and X² is independently a chlorine atom or a bromine atom,
to react with ammonia in the presence of a hydrophilic ether.

2. A process for producing 2-amino-4-trifluoromethylpyridine or a salt thereof, which comprises allowing a compound represented by the formula (II): 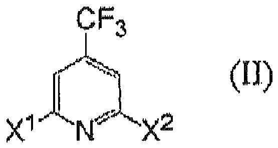 wherein each of X¹ and X² is independently a chlorine atom or a bromine atom,
to react with ammonia in the presence of a hydrophilic ether to produce a compound represented by the formula (I): 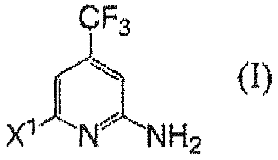 wherein X¹ is the same as X¹ in the formula (II),
and subsequently subjecting the obtained compound represented by the formula (I) to dehalogenation.

3. The method according to claim 1 or 2, wherein the hydrophilic ether is at least one member selected from the group consisting of tetrahydrofuran, 2-methyltetrahydroiuran, 3-methyltetrahydrofuran, and 1,2-dimethoxyethane.

4. The method according to claim 1 or 2, wherein the hydrophilic ether is used in an amount of from 1 to 10 times (V/W) relative to the compound of the formula (II).

5. The method according to claim 1 or 2, wherein X¹ and X⁷ are a chlorine atom.

6. An acid addition salt of 2-amino-4-trifluoromethylpyridine.
